# EUROPEAN PATENT APPLICATION

(11) **EP 4 516 347 A1**
(43) Date of publication of application: **05.03.2025**
(21) Application number: 23194650.0
(22) Date of filing: 31.08.2023
(51) Int. Cl.: A61N 1/36, B29C 65/00, H05K 3/46, A61N 1/375, A61N 1/39, A61B 5/00

(54) **PARTIAL PUSHING-IN OF CONDUCTIVE PATTERNS IN LCP**

(71) Applicant: Dyconex AG, 8303 Bassersdorf (CH)
(72) Inventor: Schmidt, Gregor, 8712 Stäfa (CH)
(74) Representative: Schulz Junghans Patentanwälte PartGmbB

(57) **Abstract**

The present invention relates to a device (1), comprising: an electrically conducting member (2) comprising a central portion (20) and an edge portion (21), the central portion (20) being integrally connected to the edge portion (21), wherein the edge portion (21) comprises a bottom side (21a), and a substrate (3) comprising a first side (3a) and a second side (3b) facing away from the first side (3a), wherein the electrically conducting member (2) is arranged on the first side (3a) of the substrate (3), wherein the edge portion (21) of the electrically conducting member (2) is embedded in the first side (3a) of the substrate (3) with the bottom side (21a) first, so that the bottom side (21a) of the edge portion (21) of the electrically conducting member (2) is arranged below a level of an adjacent surface portion (30) of the first side (3a) of the substrate (3).

## Description

The present invention relates to a device comprising an electrically conducting member arranged on a substrate as well as to a method for manufacturing such a device.

In the case of electrically conducting elements arranged on a substrate, the challenge usually arises of securing these conducting elements in such a way that detachment of the conducting elements due to mechanical forces acting on edge areas of the conducting elements is prevented.

General solutions to improve the adhesion properties of coatings, especially metallic thin films on polymeric substrates, can involve the pretreatment of the polymeric substrate (e.g. cleaning steps, mechanical roughening, plasma processes to adjust surface energies), as well as increasing the performance of the thin film process (e.g. in PVD processes) or the current density (in electrochemical processes).

Furthermore, specific adjustments of material properties and/or parameters can be made such as matching the film quality and thickness to the substrate and end-use application requirements, e.g., to avoid stress cracking or delamination. Furthermore, edge coverage of the conducting members / layers with the help of a printing process can be used.

However, the general solutions described above for optimizing adhesion properties all have the risk of quality variations due to material and process tolerances in common. These quality variations can lead to insufficient adhesion of the applied layers / conducting members. It is particularly unfavorable for the manufacturer if this is not detectable during or directly after the manufacturing process, but only occurs in external load tests or in the final application. Furthermore, partial overprinting of the coating edges with a varnish has the disadvantage that the conducting member's (e.g. electrode) surfaces are lower in profile than the varnish layer and that the varnish introduces an additional material into the structure that may not be compatible with the requirements.

There are different requirements for the adhesion of coatings in the PCB industry. In the qualification of end applications, in addition to simple peel tests, the combination with climatic preloading / soak tests is often required.

The adhesion influencing parameters such as substrate surface condition, coating thickness and coating stresses are often difficult to control due to batch and process tolerances.

Therefore, based on the above, the problem underlying the present invention is to improve the adhesion of the electrically conducting member to the surface of the substrate.

This problem is solved by a device having the features of claim 1, by a medical device having the features of claim 11, and by a method having the features of claim 12. Preferred embodiments of these aspects of the present invention are stated in the corresponding dependent claims and are described below.

According to claim 1, a device is disclosed, comprising:
- an electrically conducting member comprising a central portion and an edge portion, the central portion being integrally connected to the edge portion, wherein the edge portion comprises a bottom side, and
- a substrate comprising a first (e.g. upper) side and a second (e.g. lower) side facing away from the first side, wherein the electrically conducting member is arranged on the first side of the substrate,
wherein the edge portion of the electrically conducting member is embedded in the first side of the substrate with the bottom side ahead, so that the bottom side of the edge portion of the electrically conducting member is arranged below a level of an adjacent surface portion of the first side of the substrate.

Thus, the present invention aims to locally press in the edge portion of the respective electrically conducting member (e.g. electrode contact member), particularly by exerting a pressure and applying heat to the respective electrically conducting member.

According to a preferred embodiment of the device according to the present invention, the edge portion is arranged below a level of the central portion (e.g. embedded deeper into the first side of the substrate than the central portion.

Further, according to a preferred embodiment of the device, the edge portion is embedded such in the first side of the substrate that the edge portion comprises a section in which the edge portion is flush with the adjacent surface portion of the first side of the substrate, wherein preferably the complete edge portion is flush with the adjacent surface portion.

Furthermore, according to a preferred embodiment of the device, the edge portion is embedded such in the first side of the substrate that the edge portion comprises a section that is overlapped by a portion of the substrate (i.e. said section of the edge region is arranged below said portion of the substrate), wherein preferably the complete edge region is embedded in the substrate and overlapped by said portion of the substrate.

Further, according to a preferred embodiment of the device according to the present invention, the edge portion is a circumferential edge portion.

According to a further preferred embodiment, the electrically conducting member is an electrode contact member configured to contact tissue of a human or animal body to apply an electrical signal to said tissue. Preferably, in an embodiment, the contact member is a flat contact pad. Furthermore, according to a preferred embodiment, the contact pad comprises one of: a circular shape, a polygonal shape, an oval shape.

According to yet another preferred embodiment, the electrically conducting member is an electrically conducting track. Particularly, said conducting track can electrically connect two electronic components of an electronic circuit. Further, the electrically conducting track may also connect an electronic component to a contact pad. Further, the electrically conducting track may also connect to contact pads to one another.

According to yet another preferred embodiment of the present invention, the substrate comprises or is formed out of a thermoplastic material, such as a thermoplastic polymer. According to a preferred embodiment, the substrate comprises or is formed out of a liquid crystal polymer (LCP). According to anther embodiment, the substrate comprises or is formed out of a polyimide, polytetrafluoroethylene, or polyether ether ketone.

Within the meaning of the present invention, the term "liquid crystal polymer" is used in the meaning known to and commonly used by a person skilled in the art. A "liquid crystal polymer" refers in particular to an aromatic polymer, which has highly ordered or crystalline regions in the molten state or in solution. Non-limiting examples include aromatic polyamides such as aramid (Kevlar) and aromatic polyesters of hydroxybenzoic acid, such as a polycondensate of 4-hydroxybenzoic acid and 6-hydroxynaphthalene-2-carboxylic acid (Vectran).

According to a preferred embodiment, the substrate comprises a plurality of layers, which are stacked on top of one another in particular.

Furthermore, according to a preferred embodiment, the layers are formed from different materials. At least one layer is formed out of an LCP, preferably several or all layers are formed out of an LCP. The respective LCP is preferably one of the substances defined above.

According to an alternative embodiment, the layers are formed out of the same material, wherein preferably said material is a polymer, preferably LCP. The LCP is preferably one of the substances defined above.

According to yet another preferred embodiment, at least two layers are formed out of a different material, preferably a different LCP. These LCPs are preferably selected from the group of substances defined above. Preferably, these two different materials (e.g. LCPs) comprise a different melting temperature.

A further aspect of the present invention relates to a medical device comprising a device according to the present invention.

According to a preferred embodiment, the medical device is configured to generate an electrical signal and to apply said signal via the electrically conducting member to tissue of a human or animal body.

Furthermore, according to a preferred embodiment, the medical device can be one of: an implantable medical device, a monitoring device, a cardiac pacemaker, a neuro-stimulating device, a cardioverter defibrillator (ICD).

Yet another aspect of the present invention relates to a method for embedding an edge region of an electrically conducting member (particularly of a device according to the present invention) into a substrate, the method comprising the steps of:
- providing an electrically conducting member comprising a central portion and an edge portion, the central portion being integrally connected to the edge portion, wherein the edge portion comprises a bottom side, wherein the electrically conducting member is arranged on a substrate comprising a first (e.g. upper) side and a second (e.g. lower) side facing away from the first side, wherein the electrically conducting member is arranged on the first side of the substrate, and
- locally melting the first side of the substrate and pressing with a surface of a tool against the edge portion of the electrically conducting member to embed the edge portion in the first side of the substrate with the bottom side ahead, so that the bottom side of the edge portion of the electrically conducting member is arranged below a level of an adjacent surface portion of the first side of the substrate.

Furthermore, according to a preferred embodiment of the method according to the present invention, the tool comprises a temperature in the range between 120 °C and 400 °C upon pressing with its surface against the edge portion of the electrically conducting member, and/or wherein the surface of the tool exerts a pressure on the edge portion in the range from 0.1 N^{∗}mm⁻² to 50 N^{∗}mm⁻² upon pressing against the edge portion of the electrically conducting member.

Thus, particularly, the first side of the substrate is heated to locally melt or at least soften (i.e. the tool is heated above the Vicat softening temperature of the substrate) the first side of the substrate via the surface of the tool. The tool, particularly its surface, can be formed out of a metal or comprise a metal and can be heated using different techniques, particularly inductive heating. In addition, the tool may further comprise a hard layer on the surface comprising suitable materials such as, for example diamond like carbon, titanium nitride or titanium aluminum nitride. Alternatively or additionally, a flexible layer may be arranged between the tool and the surface, particularly in order to prevent an undesired adhesion of the substrate to the tool and/or to level unevenness's in the tool and/or the substrate.

According to a preferred embodiment of the method, the edge portion is pressed such with said surface of said tool that it becomes arranged below a level of the central portion (e.g. gets embedded deeper into the first side of the substrate than the central portion).

Further, according to a preferred embodiment of the method, the edge region is pressed with said surface of said tool to embed it in the first side of the substrate such that the edge portion comprises a section in which the edge portion is flush with the adjacent surface portion of the first side of the substrate, wherein preferably the complete edge portion is flush with the adjacent surface portion.

Furthermore, according to a preferred embodiment of the method according to the present invention, the edge portion is pressed with said surface of said tool to embed it in the first side of the substrate such that the edge portion comprises a section that is overlapped by a portion of the substrate (i.e. said section of the edge portion becomes arranged below said portion of the substrate), wherein preferably the complete edge region is overlapped by said portion of the substrate.

Particularly, according to a preferred embodiment, the surface of the tool merely contacts the edge portion or portions thereof upon pressing against the edge portion, while the central portion of the electrically conducting member is not contacted by said surface of said tool.

According to a preferred embodiment of the method, the surface is a corrugated surface. Particularly, the corrugated surface can comprise a plurality of grooves, with a ridge formed between each two neighboring grooves. Preferably the grooves / ridges extend along the edge portion of the respective electrically conducting member that is to be embedded into the substrate material.

Furthermore, the method according to the present invention can be further characterized by the features and embodiments described in conjunction with the device according to the present invention.

In the following, embodiments of the present invention as well as further features and advantages of the present invention shall be described with reference to the Figures, wherein
- Fig. 1: shows a perspective view of a substrate comprising electrical conducting members in form of contact pads thereon and a tool for pressing against edge portions of the contact pads;
- Fig. 2: shows a cross-sectional view of a substrate comprising an electrically conducting member in form of a contact pad arranged thereon and a tool for pressing against an edge portion of the contact pad; and
- Fig. 3: shows a modification of the tool shown in Fig. 2, wherein here the tool comprises a corrugated surface (A); the lower part (B) shows the edge portion of the electrically conducting member / contact pad after being pressed into the substrate.
- Fig. 4: shows a cross-sectional view of a substrate comprising an electrically conducting member in form of a contact pad arranged thereon and a first tool for pressing against an edge portion of the contact pad.
- Fig. 5: shows a cross-sectional view of a substrate comprising an electrically conducting member in form of a contact pad arranged thereon and a second tool for further pressing against the edge portion of the contact pad.

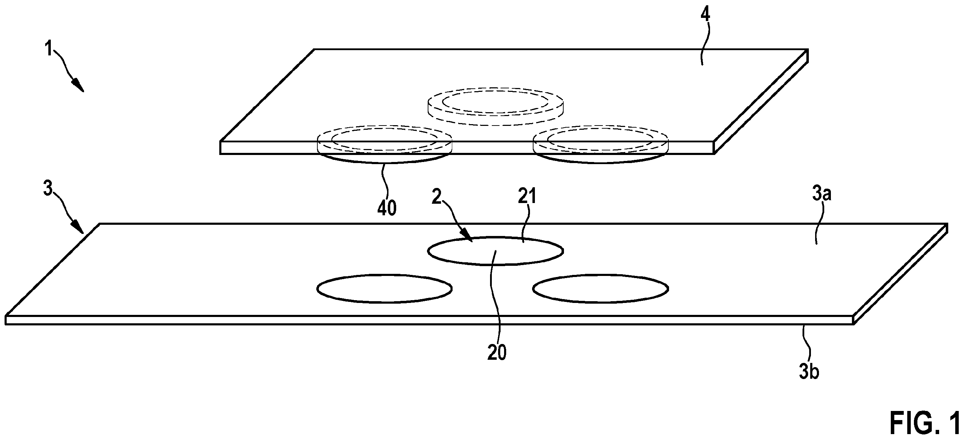

Fig. 1 shows a perspective view of an embodiment of the present invention, i.e., a device comprising a substrate 3 and at least one electrically conducting member 2, here three such members 2 in form of a flat circular contact pad 2 that may be formed out of thin film of a metal. The respective electrically conducting member 2 comprises an edge portion 21 and a central portion 20 being integrally connected to the edge portion 21. Preferably, the substrate is a thermoplastic polymeric substrate and may be formed out of at least one layer of a liquid crystal polymer (LCP). The substrate 3 may be a printed circuit board, wherein the electrically conducting members 2 may be laminated onto the substrate 3 and may by formed regarding their contour by a chemical etching process known in the prior art.

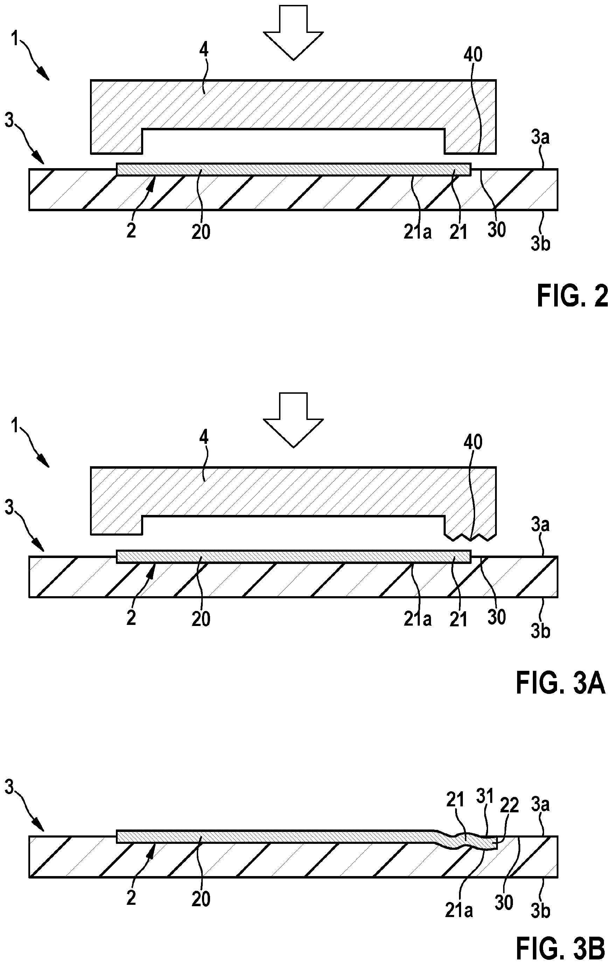

As indicated in Fig. 2, the present invention provides a method to partially indent the edge portions 21 of the electrically conducting members 2 (e.g., patterned metallic thin films) into a first side 3a of the substrate 3.

Ideally, mechanical indentation achieves better adhesion of the top portion or layer (e.g. coating) of the substrate 3 on its first side 3a to the outer edge portions 21 of the electrically conducting members 2. Preferably, the process is carried out under the application of heat applied to the respective edge portion 21 and substrate 3 to locally melt the substrate 3 in the vicinity of the edge portion 21. The structure of the tool 4, particularly of a surface 40 via which the tool 4 presses against the edge portion 21, is preferably adapted to the layout / contour of the respective electrically conducting member 2, the respective material, particularly polymer, of the substrate 3 at the first side 3a of the substrate to partially melt the substrate 3 in the region of the edge portion 21 and optimally cover the impressed substrate portion (e.g. coating).

In particular, the process is intended to be used for electrode contact pads 2 on biocompatible LCP-substrates 3 and allows the substrate 3 and electrically conducting members 2 to withstand mechanical peel tests and climatic stress cycles in qualification tests.

According to an embodiment, the substrate 3 shown in Figs. 1 to 3 may be a printed circuit board (PCB) made of an LCP with at least one substrate layer and may comprise common elements of packaging and interconnection technology, such as electrically conducting tracks, vias and said electrically conducting members 2, preferably in form of contact pads 2 on the surface / first side 3a of the substrate for contacting components or for the function of electrodes. The layers of the substrate 3 may be made of the same LCP material, or of LCP material with different properties, for example different melting points.

Particularly, as indicated in Figs. 2 and 3, the tool 4 for partial indentation of the metallic electrically conducting members 2 (e.g. pads) preferably contains 3D structures matched to the substrate 3, which are designed in such a way that during indentation the maximum pressure is present overlapping at the edge portion 21 of the respective conducting member 2.

The tool 4 is now used to press it with its surface 40 against the edge portion 21 of the respective electrically conducting member 2 and to thereby press the respective edge portion 21 into the first side 3a of the substrate 3 under pressure and temperature. The temperature is preferably selected such that the substrate 3 (e.g. LCP) melts on the first side 3a at least partially in the vicinity of the edge portion 21. The tool 4 can be used either in a separate indentation process, such as a bonding apparatus or a small press. In addition to pressure, heat can be introduced into the process, and the tool 4 may be heated above the melting temperature of the substrate 3. Alternatively, in the process of pressing in a laminating plant, the tools (press plates) could be provided with structures like the surface 40 over a large area.

According to yet another embodiment, which is indicated in Fig. 3 (A), the surface 40 of the tool 4 may be fluted in the sense that the surface 40 comprises a corrugated structure having alternating grooves and ridges to improve embedding the respective edge portion 21 into the molten substrate, ideally in a manner that the edge portion 21 comprises a section 22 that is overlapped by a portion 30 of the substrate 3. This may be achieved by suitable temperature settings, structuring of the tool 4, and material selection (e.g. LCP) allowing the substrate material to flow laterally partially over the edge portions 21. This secures the edge portion 21 against being peeled off from the substrate 3 in an optimal fashion.

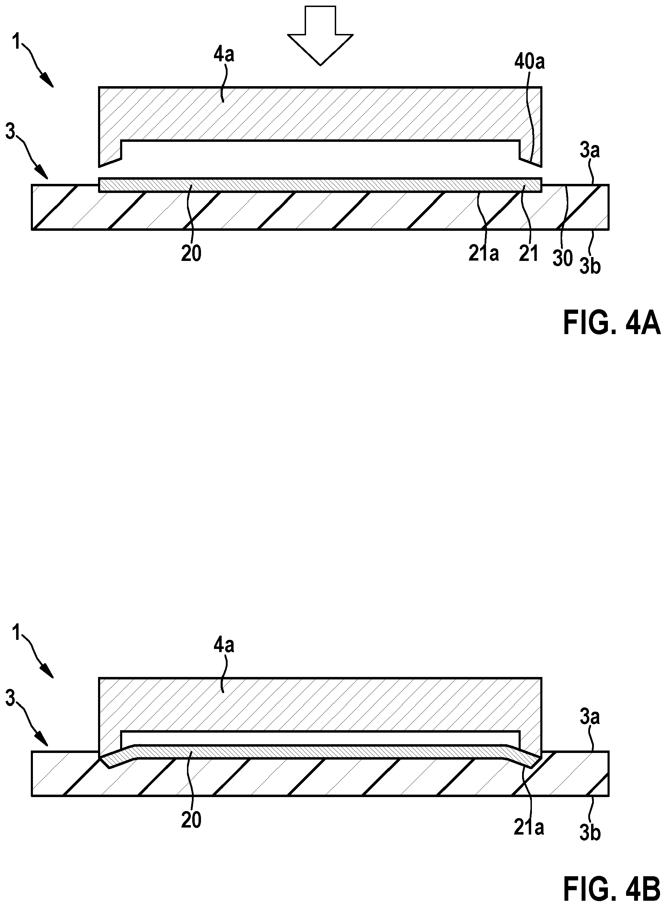

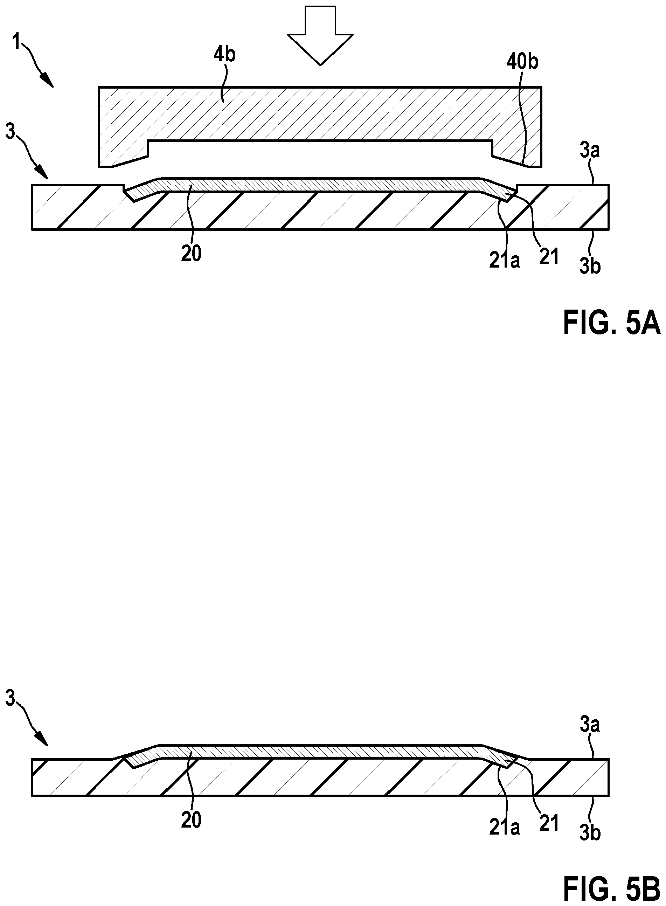

According to yet another embodiment being particularly indicated Figs. 4 and 5, the process according to the invention may comprise two steps utilizing two different tools 4a, 4b for partial indentation of the metallic electrically conducting members 2.

In a first step (Fig. 4), the edge portion 21 of the electrically conductive member 2, e.g. a contact pad, is pressed into the substrate by a first tool 4a under the application of heat, similarly to the embodiments shown Figs. 2 and 3. Here, the edge portion 21 is particularly pressed into the substrate 3 such the edge portion 21 is arranged below the surface of the first side 3a of the substrate 3, particularly by application of a sufficient first pressure or force. Particularly, the first tool 4a is dimensioned such that the edges or perimeter of the electrically conductive member 2 and the first tool substantially match to one another, particularly such that the edge portion 21 exactly is pressed into the substrate 3. Particularly, the surface 40a of the first tool being in contact with electrically conductive member 2 and/or the substrate 3 is inclined.

In a second step (Fig. 5), the edge portion 21 of the electrically conductive member 2 once again is pressed into the substrate by a second tool 4b under the application of heat. Here, the second tool 4a is dimensioned such that the surface 40b of the second tool 4b contacts the already indented edge portion 21 and an area of the first side 3a of the substrate 3 bordering the edge portion 21. Also here, the surface 40b of the first tool being in contact with electrically conductive member 2 and/or the substrate 3 is preferably inclined. Particularly, the edge portion 21 is pressed into the substrate 3 by the second tool with a second pressure or force, being particularly smaller than the first pressure or force. As a result, a portion of the substrate 3 flows over a part of the edge portion and thereby encapsulate that part. Consequently, the adhesion of the electrically conductive member 2 may be further enhanced.

The present invention advantageously allows improving the adhesion of an electrically conducting member (e.g. contact pad) 2. Especially in peel tests and also in stresses caused by exposure of the substrate to moisture / or in liquids, the edges of the pads are the main point of attack. If the edge portions are pressed into the substrate and/or are partially covered by substrate material, this point of attack is reduced.

## Claims

1. A device (1), comprising:
- an electrically conducting member (2) comprising a central portion (20) and an edge portion (21), the central portion (20) being integrally connected to the edge portion (21), wherein the edge portion (21) comprises a bottom side (21a), and
- a substrate (3) comprising a first (e.g. upper) side (3a) and a second (e.g. lower) side (3b) facing away from the first side (3 a), wherein the electrically conducting member (2) is arranged on the first side (3a) of the substrate (3),
wherein the edge portion (21) of the electrically conducting member (2) is embedded in the first side (3a) of the substrate (3) with the bottom side (21a) first, so that the bottom side (21a) of the edge portion (21) of the electrically conducting member (2) is arranged below a level of an adjacent surface portion (30) of the first side (3a) of the substrate (3).

2. The device according to claim 1, wherein the edge portion (21) is arranged below a level of the central portion (20).

3. The device according to claim 1 or 2, wherein the edge region (21) is embedded such in the first side (3a) of the substrate (3) that the edge portion (21) comprises a section in which the edge portion (21) is flush with the adjacent surface portion (30) of the first side (3a) of the substrate (3).

4. The device according to one of the preceding claims, wherein the edge portion (21) is embedded such in the first side (3a) of the substrate (3) that the edge portion (21) comprises a section (22) that is overlapped by a portion (30) of the substrate (3).

5. The device according to one of the preceding claims, wherein the edge portion (21) is a circumferential edge portion (21).

6. The device according to one of the preceding claims, wherein the electrically conducting member (2) is an electrode contact member configured to contact tissue of a human or animal body to apply an electrical signal to said tissue.

7. The device according to claim 6, wherein the electrode contact member (2) is a flat contact pad.

8. The device according to one of the claims 1 to 5, wherein the electrically conducting member is an electrically conducting track.

9. The device according to one of the preceding claims, wherein the substrate (3) comprises or is formed out of a thermoplastic material.

10. The device according to one of the preceding claims, wherein the substrate (3) comprises or is formed out of a liquid crystal polymer (LCP), a polyimide, polytetrafluoroethylene, or polyether ether ketone.

11. A medical device, comprising a device (1) according to one of the preceding claims.

12. A method for embedding an edge portion (21) of an electrically conducting member (2) into a substrate (3), the method comprising the steps of:
- providing an electrically conducting member (2) comprising a central portion (20) and an edge portion (21), the central portion (20) being integrally connected to the edge portion (21), wherein the edge portion (20) comprises a bottom side, wherein the electrically conducting member (2) is arranged on a substrate (3) comprising a first side (3a) and a second side (3b) facing away from the first side (3a), wherein the electrically conducting member (2) is arranged on the first side (3a) of the substrate (3), and
- locally softening or melting the first side (3a) and pressing with a surface (40, 40a, 40b) of a tool (4, 4a, 4b) against the edge portion (21) of the electrically conducting member (2) to embed the edge portion (21) in the first side (3a) of the substrate (3) with the bottom side (21a) first, so that the bottom side (21a) of the edge portion (21) of the electrically conducting member (2) is arranged below a level of an adjacent surface portion (30) of the first side (31) of the substrate (3).

13. The method according to claim 12, wherein the tool (4, 4a, 4b) comprises a temperature in the range between 120 °C and 400 C upon pressing with its surface (40) against the edge portion (21) of the electrically conducting member (2).

14. The method according to claim 12 or 13, wherein the surface (40) of the tool (4, 4a, 4b) exerts a pressure on the edge portion (21) in the range from 0.1 N^{∗}mm⁻² to 50 N^{∗}mm⁻² upon pressing against the edge portion (21) of the electrically conducting member (2).

15. The method according to any one of claims 12 to 14, wherein the surface (40) is a corrugated surface (40).
